**Europäisches Patentamt**

**European Patent Office**

**Office européen des brevets**

(19)

(11) Veröffentlichungsnummer: **0 119 480**

**B1**

(12) **EUROPÄISCHE PATENTSCHRIFT**

(45) Veröffentlichungstag der Patentschrift:
25.01.89

(51) Int. Cl.⁴: **A 61 K 9/16**

(21) Anmeldenummer: **84101627.2**

(22) Anmeldetag: **16.02.84**

(54) Sphärische Einkristalle für pharmazeutische Zwecke.

(30) Priorität: **23.02.83 DE 3306250**

(43) Veröffentlichungstag der Anmeldung:
**26.09.84 Patentblatt 84/39**

(45) Bekanntmachung des Hinweises auf die Patenterteilung:
**25.01.89 Patentblatt 89/4**

(84) Benannte Vertragsstaaten:
**AT BE CH DE FR GB IT LI NL SE**

(56) Entgegenhaltungen:
**FR-M-7 932**
**NL-C-137 432**

**CHEMICAL ABSTRACTS, Band 99, Nr. 2, 11. Juli 1983, Seite 299, Nr. 10762h, Columbus, Ohio, US; Y. KAWASHIMA u.a.: "An experimental study of the kinetics of the spherical crystallization of sodium theophylline monohydrate" & POWDER TECHNOL. 1983, 34(2), 255-60**
**CHEMICAL ABSTRACTS, Band 86, Nr. 10, 7. März 1977, Seite 356, Nr. 60544r, Columbus, Ohio, US; & JP - A - 76 113 813 (TAKASHI HIRAIWA) 07.10.1976**
**J. Appl. Biotechnol 1978, 28, 663-667, Sarig et al.**

(73) Patentinhaber: **BASF Aktiengesellschaft, Carl-Bosch- Strasse 38, D-6700 Ludwigshafen (DE)**

(72) Erfinder: **Pich, Claus Hinrich, Dr., An der Duene 3, D-2082 Moorrege (DE)**
Erfinder: **Moest, Thomas, Dr., An der Duene 9, D-2082 Moorrege (DE)**

## Beschreibung

Die Erfindung betrifft sphärische Einkristalle pharmazeutischer Wirk- oder Hilfsstoffe mit Durchmessern von 0,1 bis 3 mm zur Verwendung bei der Herstellung von festen pharmazeutischen Produkten.

Zur Herstellung von festen, oralen Arzneimitteln sind staubfreie sphärische Partikeln mit engem Kornband (Streubreite des Durchmessers) von besonderem Interesse. Sie haben eine gute Rieselfähigkeit und sind deshalb sehr genau über das Volumen zu dosieren, was z. B. für das Füllen von Hartgelatinekapseln von großer Bedeutung ist. Wegen der kugelförmigen Oberfläche existieren nur geringe Kontaktflächen, so daß ein Verklumpen untereinander praktisch nicht auftritt. Die Gefahr der Entmischung, wie sie bei Granulaten mit breitem Kornband immer befürchtet werden muß, ist erheblich reduziert.

Eine große Bedeutung kommt der Umhüllung dieser Partikeln zu. Im Gegensatz zu scharfkantigen Kristallen und unregelmäßig geformten Agglomeraten lassen sie sich gleichmäßig und ohne Verklebungen mit den unterschiedlichsten Überzügen versehen. Außerdem benötigt man weniger Hüllsubstanz, um die gewünschten Eigenschaften wie Geschmacksneutralität, Magensaftresistenz, Retardierung oder Stabilisierung zu erreichen.

Die unbehandelten oder in der beschriebenen Weise nachbehandelten, d.h. umhüllten, abgerundeten Körner lassen sich vorteilhaft direkt oder nach Abfüllung in Hartgelatinekapseln applizieren.

Für die Herstellung solcher sphärischen, auch "Pellets" genannten Teilchen sind dem Galeniker im wesentlichen zwei Verfahren bekannt: Man kann runde Partikeln durch Agglomeration feiner kantiger Primärteilchen, die anschließend durch plastische Verformung oder Abrieb mechanisch gerundet werden, erzeugen. Ein anderes Verfahren ist die Aufdragierung von kantigen Starterkernen mit Kleblösung und Puder bis zur Ausrundung.

Beide Verfahren haben Nachteile: Eine gezielte Steuerung der Partikelgröße ist meist nicht möglich. Es entsteht ein breites Kornband; Über- und Unterkorn müssen abgesiebt werden. Außerdem sind die Partikeln porös und oft wenig fest, so daß sie druckempfindlich sind und durch Abrieb leicht unerwünschter Staub entsteht. Ferner können nach beiden Verfahren keine reinen Substanzen hergestellt werden, da zusätzliche Stoffe, wie Kleb- und Füllmittel, eingearbeitet werden müssen.

Der Erfindung lag daher die Aufgabe zugrunde, hier Abhilfe zu schaffen, also die geschilderten Nachteile des Standes der Technik zu vermeiden, d.h., in einfacher Weise erzeugte sphärische, druck- und abriebfeste, nicht poröse Partikeln mit gezielter Korngröße und enger Korngrößenverteilung und hoher Reinheit bei der Herstellung fester pharmazeutischer Produkte einzusetzen.

Die Lösung dieser Aufgabe besteht in der Verwendung von sphärischen Einkristallen von pharmazeutischen Wirk- und Hilfsstoffen mit Durchmessern von 0,1 bis 3, vorzugsweise 0,5 bis 2 mm bei der Herstellung von festen pharmazeutischen Produkten.

Die Herstellung sphärischer Einkristalle, vor allem von Kaliumchlorid, ist an sich bekannt (Dissertation Beer, Technische Universität München, vom 19.01.81; S. Sarig, N. Eidelmann, A. Glasner und J.A. Epstein, J. of Appl. Chem. Biotechnol. 28 (1978) 10, 663 - 667; M.A. Belyshev, G.P. Baranov, V.A. Postnikov und V.A. Ryabkov, Khim. Prom. 6, (1977), 455 - 459; NL 137 432). Sie wurden jedoch nicht zur Verwendung in der pharmazeutischen Industrie vorgeschlagen, obwohl, wie oben dargelegt, ein dringender Bedarf vorlag.

Sphärisch im Sinne der Erfindung sind nicht nur streng kugelförmige Teilchen, sondern auch solche Teilchen ohne ebene Flächen und ohne scharfe Kanten, deren größter zum kleinsten Durchmesser sich maximal wie 3 : 1, vorzugsweise kleiner als 1,5 : 1 verhält. Die Angabe der Korngröße bezieht sich in solchen Fällen auf den kleinsten Durchmesser.

Der Begriff "pharmazeutischer Wirkstoff" bedarf keiner Erläuterung. Das sind, wie das Wort sagt, Stoffe, die eine pharmazeutische Wirkung zeigen, und deren unerwünschten Nebenwirkungen dabei gering genug sind, daß ihr Einsatz als Heilmittel in Betracht kommt.

Der Begriff "löslicher, kristalliner, pharmazeutischer Hilfsstoff" umfaßt u.a. beispielsweise Füllstoffe wie Lactose, Ca-Phosphat, Kochsalz, Harnstoff, Mannit; die Komponenten von Brausemischungen wie Natriumbicarbonat und Zitronensäure oder Weinsäure; Geschmacksverbesserer wie Zucker (z. B. Glucose, Sacccharose), Zuckeraustauschstoffe (z. B. Sorbit, Xylit) und synthetische Süßstoffe (z. B. Saccharin und Cyclamat).

Der Begriff "pharmazeutischer Hilfsstoff" ist z. B. in folgenden Werken näher erläutert: H.P. Fiedler, Lexikon der Hilfsstoffe für Pharmazie, Kosmetik und angrenzende Gebiete, 2. Auflage, Editio Cantor K.G., D-7 960 Aulendorf, 1981, Band I und II; Hagers Handbuch der Pharmazeut. Praxis, 4. Aufl., Berlin - Heidelberg - New York, Springer - Verl., 1967 - 1980, Bd. VII, Teil B; sowie in den üblichen Pharmakopöen (DAB, PhEur, BP, USP usw.). Es versteht sich von selbst, daß Voraussetzung für die Eignung einer Substanz zum erfindungsgemäßen Einsatz deren Fähigkeit zu kristallisieren ist. Polymere kommen daher nicht in Betracht. Andererseits sind grundsätzlich alle kristallisierenden, insbesondere die leicht (grob) kristallisierenden Verbindungen einsetzbar. Es versteht sich ferner von selbst, daß die Wirksubstanzen im Mundspeichel oder mindestens im Magen-Darm-Trakt für den jeweiligen therapeutischen Zweck ausreichend löslich sein müssen.

Feste pharmazeutische Produkte im Sinne der Erfindung sind Granulate, Tabletten, Filmtabletten, Brausetabletten, Dragees und Hartgelatine-Steckkapseln. Ihre Zubereitung ist dem Fachmann geläufig und ebenfalls z. B. in den oben genannten Standardwerken beschrieben. Die erfindungsgemäß einzusetzenden sphärischen Einkristalle stellen Pellets dar und können als solche unmittelbar oder nach dem Überziehen der Wirkstoffpartikeln mit Hilfsstoffen oder von Hilfsstoffpartikeln mit Wirkstoffen, gegebenenfalls in mehreren wechselnden Schichten, in den Handel kommen, sie können aber auch durch Tablettieren (Verpressen mit Bindemittel) oder durch Abfüllen in Kapseln in eine handliche, d.h. bequem und exakt dosierbare Form gebracht werden.

Die Herstellung der erfindungsgemäß zu verwendenden sphärischen Einkristalle erfolgt in an sich bekannter Weise (vgl. die eingangs genannte Literatur) in hochtourigen Rührwerks- oder Leitrohrkristallisatoren durch Abkühlen oder Einengen aus wäßrigen, organischen oder organisch-wäßrigen Lösungen. Entscheidend ist eine ausreichende Bewegung der Kristallteilchen dergestalt, daß das geordnete flächenzentrierte Wachstum der Kristalle durch die Relativbewegung von Teilchen gegen die Lösung gestört wird. Im einfachsten Fall wird kräftig gerührt. Die erforderliche Rührgeschwindigkeit hängt von der Geometrie von Rührer und Gefäß ab. Sie liegt im allgemeinen in der Größenordnung von 50 bis 1000, vorzugsweise 100 bis 600 Umdrehungen pro Minute. Ferner ist es erforderlich, die gesättigte Lösung mit Saatkristallen zu impfen. Diese sind zweckmäßig feingemahlen, d.h. die Korngröße liegt unter 300, vorzugsweise unter 100 μm Durchmesser. Die Lösung darf zum Zeitpunkt der Zugabe der Saatkristalle und während ihres Wachstums stets nur minimal übersättigt sein. Damit ist die Einhaltung eines engen Kornbandes gewährleistet. Die minimale Übersättigung wird durch langsames Abkühlen (nicht mehr als 50, vorzugsweise weniger als 10, insbesondere weniger als 5 K/h) bzw. durch entsprechend langsames Eindampfen der Lösung erreicht.

Die Herstellung kann diskontinuierlich und kontinuierlich erfolgen. Die Ergebnisse von Versuchen zum Einfluß der Rührintensität, des Feststoffgehaltes der Kristallsuspension und der Verweilzeit können von diskontinuierlicher auf kontinuierliche Verfahrensweise übertragen werden. Wirtschaftlich vorteilhaft wird die sphärische Kristallisation mit der ohnehin meist notwendigen Reinigung der Substanzen durch Umkristallisation verbunden.

Die Korngröße wird beim kontinuierlichen Verfahren vorwiegend über die Verweilzeit eingestellt. Bei diskontinuierlicher Arbeitsweise hängt die Korngröße neben der Kristallisierzeit von der Zahl der Saatkristalle ab. Bei einem Korndurchmesser der Saatkristalle unter 30 μm liegt das Gewichtsverhältnis Saatkristalle zu auszukristallisierender Substanz im allgemeinen in der Größenordnung von 1 : 1 000 bis 1 : 1 000 000, vorzugsweise bei 1 : 10 000 bis 1 : 100 000. Man erhält harte, druck- und abriebfeste, nicht poröse sphärische Partikeln (Einkristalle). Es handelt sich um reine Substanzen ohne Beimengungen.

Die Vorteile der sphärischen Form der Partikeln kommen ganz besonders dann zum Tragen, wenn man sie mit einem Überzug versieht. Im Vergleich zu kantigen Partikeln wird der Überzug sehr gleichmäßig, man kommt mit wesentlich weniger Überzugsmaterial aus und der angestrebte Effekt (z. B. Magensaftresistenz, eine gesteuerte z. B. linear-verzögerte Wirkstoff-Freisetzung, gegebenenfalls gekoppelt mit einer Mehrphasenwirkung des Dragees, oder auch nur die Verdeckung eines unangenehmen Geschmacks) wird besser und zuverlässiger erreicht.

Die Art der in Betracht kommenden Überzugsmaterialien ist in der zu den pharmazeutischen Hilfsstoffen genannten Literatur beschrieben und dem Fachmann geläufig.

Infolge der großen Stabilität gegenüber Verformungskräften können aus den so erzeugten sphärischen Partikeln Tabletten gepreßt werden, die nach oraler Applikation im Magen zunächst in die ursprünglichen, unbeschädigten Partikeln zerfallen. Dies ist insbesondere bei retardierten und magensaftresistenten Tabletten von Bedeutung, da diese infolge ihrer Größe häufig den Pylorus nicht passieren und im Magen zurückgehalten werden. Somit können neben Steckkapseln auch Tabletten für eine medizinisch erwünschte Multi-Unit-Dose-Applikation eingesetzt werden.

Die Erfindung wird in den Beispielen näher erläutert.

Bei den Beispielen wurde auf die Ausbeute nicht geachtet. Bei kontinuierlicher Arbeitsweise und reinem Ausgangsprodukt ist sie quantitativ, ansonsten hängt sie von der Reinheit des Ausgangsmaterials und dem gewünschten Reinheitsgrad des Endproduktes sowie bei diskontinuierlicher Verfahrensweise vom Arbeitsaufwand ab und liegt zwischen 10 und 100, vorzugsweise zwischen 50 und 100 %.

**Beispiel 1**

a) In einem handelsüblichen 50-l-Rührkessel mit Propellerrührer und Doppelmantel wurden 40 l einer auf 40°C erwärmten, gesättigten Lösung von Kaliumchlorid in Wasser intensiv gerührt.

Nach Zugabe von 50 mg Kaliumchlorid-Kristallkeimen mit einer Korngröße kleiner 30 μm ließ man mit einer Kühlrate von 3 K/h unter stetigem Rühren mit einer Drehzahl von n = 300 min⁻¹ auf 20°C abkühlen.

Das entstandene Kristallisat wurde mit wenig kaltem Wasser zur Verhinderung einer Sekundärkristallisation verdünnt, sofort über eine Nutsche abgesaugt, mit wenig kaltem Wasser nachgewaschen und im Wirbelbetttrockner mit einer Zulufttemperatur von 60° C getrocknet.

Das Produkt bestand aus farblosen sphärischen Kristallen mit einem Korngrößenbereich von ca. 0,4 bis ca. 1,2 mm Durchmesser, wobei ein ausgeprägtes Maximum der Häufigkeitsverteilung bei 0,7 mm lag.

b) Die so erhaltenen sphärischen Kaliumchlorid-Kristalle wurden in einem Wirbelschichtsprühgranulator kontinuierlich mit einer ethanolischen Ethylcelluloselösung überzogen, deren Konzentration 6,5 % G/G betrug. Der spezifische Viskositätswert der Ethylcellulose betrug 10 mPas. Die Polymerenlösung enthielt als Weichmacher 20 %, bezogen auf das Polymerengewicht, an Dibutylphthalat. Die Gesamtmenge des Hüllpolymeren betrug 7 %, bezogen auf das überzogene Kaliumchlorid. Die Wirbelschichtlackierung wurde so gesteuert, daß die Produkttemperatur im Bereich von 28 bis 30° C lag.

Die so retardierten sphärischen Kaliumchloridpellets wurden mit 0,5 % hochdispersem Siliciumdioxid gemischt. Sie konnten sehr leicht und genau in üblichen Geräten in Haltgelatine-Steckkapseln abgefüllt werden.

Tabelle 1 zeigt eine Gegenüberstellung der Kaliumchlorid-Freisetzungen aus unterschiedlichen, retardierten Partikeln.

A beruhte auf den erfindungsgemäß einzusetzenden, wie geschildert hergestellten und beschichteten sphärischen Pellets mit 7 % Lack.

B enthielt übliches kubisches Kaliumchlorid mit entsprechend gewählter Korngrößenverteilung. Es wurde nach Rezeptur und Verfahren wie A mit Ethylcellulose beschichtet.

C entsprach B, jedoch betrug die Lackmenge 13 Gew. %.

T gibt die theoretische Gerade einer exakten Freisetzung nullter Ordnung wieder.

**Tabelle 1** Freisetzungen in %

Die Freisetzungen wurden gemäß den Angaben der USP XX für die Paddle-Methode bestimmt.

|  | Zeit (h) | | | |
| --- | --- | --- | --- | --- |
| Formulierung | 1 | 2 | 4 | 8 |
| A | 21 | 39 | 64 | 95 |
| B | 49 | 72 | 93 | 97 |
| C | 30 | 48 | 63 | 84 |
| T | 12,5 | 25 | 50 | 100 |

Der Vergleich zeigt, daß die erfindungsgemäß zu verwendenden sphärischen Partikeln den Idealverhältnissen einer Freisetzung nullter Ordnung relativ nahe kommen, wogegen das konventionell aufbereitete Kaliumchlorid erhebliche Abweichungen zeigt. Auch durch Variation der Lackmenge kann kubisches Kaliumchlorid nicht auf das hohe Retardierungs-Niveau der sphärischen Kristallprodukte gebracht werden.

c) Eine Mischung aus dem gemäß (a) erhaltenen retardierten, spärischen Kaliumchlorid (65 Gewichtsteile), direkttablettierbarem Calciumhydrogenphosphatdihydrat (15), mikrokristalliner Cellulose (12,5), Natriumstärkeglykolat (7) und Magnesiumstearat (0,5) in den angegebenen Anteilen wurde auf einer üblichen Rundläufer-Tablettenpresse zu 1000 mg schweren Tabletten im Format 18 x 8 mm verpreßt. Es entstanden Preßlinge mit einer Bruchfestigkeit von 70 bis 90 H (Schleuniger-Testgerät, und einem Variationskoeffizienten $V_{rel}$ des mittleren Tablettengewichtes von kleiner 1 %. Die Zerfallszeit lag unter 2 Minuten. Die Freisetzung von Kaliumchlorid aus den Retardpellets der schnell zerfallenden Tabletten (A in Tabelle 2) wich nur geringfügig von den in Tabelle 1 angegebenen Werten für nichtverpreßte, erfindungsgemäße Pellets ab.

Beim vergleichsweisen Tablettieren von retardiertem, kubischem Kaliumchlorid entsprechend B in Tabelle 1 in einer identischen Tablettenrezeptur entstanden Preßlinge mit einer maximalen Bruchfestigkeit von 70 N (Schleuniger) und einer signifikant größeren Gewichtsschwankung von $V_{rel}$ = 1,6 %.

Die Kaliumchlorid-Freisetzung aus den Pellets der zerfallenen Tabletten (B in Tabelle 2) ist gegenüber dem zugrundeliegenden retardierten kubischen Kaliumchlorid (B in Tabelle 1) nochmals deutlich beschleunigt.

**Tabelle 2** Freisetzen in %

|  | Zeit (h) | | | |
| --- | --- | --- | --- | --- |
| Formulierung | 1 | 2 | 4 | 8 |
| A | 24 | 44 | 67 | 95 |
| B | 61 | 80 | 97 | 98 |

Die erfindungsgemäß zu verwendenden retardierten, sphärischen Kaliumchloridpellets führten somit auf einfachstem Wege zu Tabletten, die eine unter medizinischen Aspekten wunschgemäße Kaliumchloridfreisetzung gewährleisten. Konventionell verarbeitetes Kaliumchlorid dagegen führte unter den angegebebenen Bedingungen nur zu Tabletten mit minimalem, inakzeptablen Retardeffekt. Eine graduelle Verbesserung war nur unter erhebliche Mehraufwand zu erreichen.

**Beispiel 2**

In einem vakuumdichten 6-l-Leitrohrkristallisator mit den Dimensionsverhältnissen d : D : H = 0,6 : 1 : 2, (d = Leitrohrdurchmesser, D = Behälterdürchmesser, H = Höhe des Behälters), ausgestattet mit Schrägblattrührer und

Doppelmantel, wurden 5 l einer auf 50°C erwärmten, gesättigten Lösung von Eisen-II-sulfat so intensiv gerührt, daß ein stetiger vertikaler Kreislauf um das Leitrohr gewährleistet war (n = ca. 200 min⁻¹).

Nach Zugabe von 10 mg Eisen-II-sulfat-Kristallkeimen mit einer Korngröße kleiner 30 µm legte man langsam Vakuum an und stellte dieses so ein, daß zunächst ca. 100 ml/h Wasser in einer Kühlfalle auskondensiert wurden. Parallel zu einer weiteren Erniedrigung des Druckes wurde die Temperatur mit einer Rate von 20 K/h stetig auf 0°C gesenkt.

Das entstandene Kristallisat wurde über eine Nutsche abgesaugt, mit wenig Wasser von 0°C nachgewaschen und in einem Vakuumtrockenschrank bei 50°C und 20 mbar getrocknet. Das Produkt bestand aus hellgrünlichen, sphärischen Kristallen mit einem Korngrößenbereich von 0,5 bis 1,3 mm Durchmesser, wobei ein deutliches Maximum der Häufigkeitsverteilung bei 0,8 mm lag.

Das so erhaltene sphärische Eisen II-sulfat wurde in einem Wirbelschichtsprühgranulator kontinuierlich mit einer Lösung von Hydroxypropylmethylcellulosephthalat in Isopropanol-Methylenchlorid 3 : 7 überzogen. Die Konzentration der Lösung betrug 7 % G/G. Die Gesamtmenge des Hüllpolymeren betrug 12 % G/G, bezogen auf das überzogene Eisen-II-sulfat. Der Polymerenlösung wurden als Weichmacher 20 % G/G Dibutylphthalat, bezogen auf das Polymerenmaterial, zugesetzt.

Die Wirbelschichtlackierung wurde so gesteuert, daß die Produkttemperatur im Bereich von 26 bis 28°C verweilte.

Die magensaftresistenten, sphärischen Eisen-II-sulfat-Pellets wurden mit 0,5 % hochdispersem Siliciumdioxid gemischt. Sie konnten sehr leicht und genau in üblichen Geräten in Hartgelatine-Steckkapseln abgefüllt werden.

Die Magensaftresistenz wurde nach der Vorschrift der Ph. Eur. geprüft.

Der Vorteil der erfindungsgemäß hergestellten magensaftresistenten Eisen-II-sulfat-Pellets wird offensichtlich bei einem Vergleich mit entsprechenden Pellets, die nach einem konventionellen Verfahren hergestellt wurden (Vergleichsversuch 1).

**Vergleichsversuch 1**

Feinkristallines Eisen-II-sulfat wurde mit einer 10-%-igen wäßrigen Lösung von Hydroxypropylmethylcellulose (spezifischer Viskositätswert 6 mPas) in einem Dragierkessel soweit agglomeriert und geformt, bis runde Granulatteilchen ausgebildet waren (vgl. W.A. Ritschel, Die Tablette, Editio Cantor K.G., D-7960 Aulendorf, 1966, S. 212 und 213). Nach der Trocknungsphase wurde das Granulat auf den Korngrößenbereich 0,3 bis 1,19 mm in zwei Durchgängen abgesiebt. Die Ausbeute dieser Fraktion betrug 63 %. Größere Agglomerate, die in hohem Anteil bis zu einer Größe von maximal 10 mm Durchmesser entstanden waren, mußten wieder zerkleinert und zusammen mit dem Feinanteil unter 0,3 mm in einem zusätzlichen Arbeitsgang neu agglomeriert werden.

Die entstandenen Pellets hatten unregelmäßige, nur annähernd kugelige Gestalt mit poröser Struktur. Der Gehalt an Eisen-II-sulfat betrug 94 %.

Eine analoge magensaftresistente Lackierung wie in Beispiel 2 beschrieben, mit 12 % G/G Hydroxypropylmethylcellulosephthalat, bezogen auf das Gesamtgewicht, durchgeführt in einer Wirbelschichtanlage, ergab kein magensaftresistentes Produkt. Erst bei einem Anteil von 16 % Lack war die Prüfung auf Magensaftresistenz gemäß Ph. Eur. positiv.

Die Schüttdichte, bestimmt nach DIN 53 468, war mit 0,65 g/ml um 14,5 % niedriger als bei dem erfindungsgemäß erhaltenen Produkt in Beispiel 2 (0,76 g/ml).

Die dosierbare Eisen-II-sulfatmenge pro Volumeninhalt war somit bei den erfindungsgemäß verwendeten sphärischen Eisen-II-sulfat-Partikeln mit magensaftresistenter Lackierung insgesamt um ca. 23 %

$$( = 100 - 0,94 \cdot \frac{100-16}{100-12} \cdot \frac{0,65}{0,76} \cdot 100)$$

größer als bei konventionell hergestellten magensaftresistenten Eisen-II-sulfat-Pellets.

In einem vergleichenden Kapselfüllversuch mit Hartgelatine-Steckkpseln der Größe 00 wurde dieses Ergebnis bestätigt. Das Verhältnis der abgefüllten - umgerechneten - Eisen-IIsulfatmengen betrug im Mittel 628 zu 486 mg. Die erfindungsgemäßen Eisen-II-sulfat-Partikeln ergaben also einen effektiven Dosiervorteil in Höhe von 22,6 %.

**Beispiel 3**

In einem handelsüblichen 50-l-Rührkessel mit Propellerrührer und Doppelmantel wurden 40 l einer auf 45°C erwärmten, gesättigten Lösung von Zitronensäure in Ethanol intensiv gerührt.

Nach Zugabe von 100 mg Zitronensäure-Kristallkeimen mit einer Korngröße kleiner 30 µm ließ man langsam mit einer Kühlrate von 6 K/h unter stetigem Rühren mit einer Drehzahl von n = 300 min⁻¹ auf 20°C abkühlen.

Das entstandene Kristallisat wurde mit wenig kaltem Ethanol verdünnt, sofort über eine Nutsche abgesaugt, mit wenig kaltem Ethanol nachgewaschen und im Wirbelbetttrockner mit einer Zulufttemperatur von 60°C getrocknet.

Das Produkt bestand aus farblosen sphärischen Kristallen mit einem Korngrößenbereich von 0,3 bis 1,0 mm, wobei ein ausgeprägtes Maximum der Häufigkeitsverteilung bei 0,5 mm lag.

Eine Mischung aus den so erhaltenen sphärischen Zitronensäurepartikeln mit Kaliumhydrogencarbonat, direkttablettierbarem Rohrzucker (Instantzucker) und Polyethylenglykol 6000 im Gewichtsverhältnis 1 : 1 : 1 :0,1 wurde auf einer Rundläufer-Tablettenpresse zu 7,75 g schwere Tabletten mit einem Durchmesser von 28 mm direktverpreßt. Man erhielt Brausetabletten mit einer Bruchfestigkeit von über 90 N (Schleuniger-Tester; Test unmittelbar nach Herstellung), einem Abrieb von 0,5 % (Roche Friabilator, ca. 100 g, 300 Umdrehungen) und einem Variationskoeffizienten für das mittlere Tablettengewicht von 0,7 %. Die Zerfallzeit gemäß Ph. Eur. lag unter 3 min.

Die bedeutenden Vorteile der Brausetablettenrezeptur, enthaltend die erfindungsgemäße, sphärische Zitronensäure, werden deutlich im Vergleich mit den Produkteigenschaften von Brausetabletten, die auf gewöhnlicher, handelsüblicher Zitronensäure mit einer mittleren Korngröße von ebenfalls 0,5 mm basieren (Vergleichsversuch 2).

## Vergleichsversuch 2

Bei identischen übrigen Rezepturbestandteilen und identischem Mischen und Verpressen erhielt man in diesem Fall Brausetabletten mit einer Bruchfestigkeit von lediglich 50 bis 70 N, einem Abrieb von 2,8 % und einem Variationskoeffizienten für das mittlere Tablettengewicht von 1,9 %. Die Zerfallszeit betrug bei exakter Einhaltung der Methodik der Ph. Eur. teilweise über 8 min und entsprach damit nicht mehr dem geforderten Limit von 5 min. Es verblieben über diesen langen Zeitraum Partikelverbände mit inkorporierter Zitronensäure, die eine ungewöhnlich schlechte Löslichkeit aufwiesen.

Der Einsatz von sphärischer Zitronensäure bei der Herstellung von Brausetabletten führt also nicht nur zu einer Verbesserung der mechanischen Tabletteneigenschaften, sondern er ermöglicht in diesem Fall sogar erst die Erfüllung der Forderungen des Arzneibuches.

## Beispiel 4

In einem handelsüblichen vakuumfesten 50-l-Rührkessel mit Propellerrührer und Doppelmantel wurden 40 l einer auf 60°C temperierten gesättigten wäßrigen Lösung von Magnesiumsulfat intensiv gerührt (n = 300 min⁻¹).

Nach Zugabe von 50 mg Magnesiumsulfat-Kristallkeimen mit einer Korngröße kleiner 30 μm legte man vorsichtig Vakuum an, bis die Lösung schwach siedete. Bei der konstanten Temperatur von 70°C wurde das Vakuum dann so eingestellt, daß in einem Kondensator eine stündliche Wassermenge von 2 l abgeschieden wurde. Nach 10 Stunden wurde der Prozeß abgebrochen, das entstandene Kristallisat sofort über eine Nutsche abgesaugt und mit wenig Wasser von 70°C nachgewaschen. Die Trocknung erfolgte in einem Schaufeltrockner bei 120°C.

Das Produkt bestand aus farblosen sphärischen Kristallen mit einem Korngrößenbereich von 0,5 bis 2 mm. Das Maximum der Häufigkeitsverteilung lag bei 1,0 mm.

Das so erhaltene sphärische Magnesiumsulfat wurde in einer rotierenden Lochtrommel (Accela Cota 24" der Fa. Manesty, Liverpool mit 0,3 mm laserstrahlgelochter Trommel) mit einer auf 40°C erwärmten 20-%-igen G/G wäßrigen Lösung von Hydroxypropylmethylcellulose (spezifischer Viskositätswert 3 mPas) mit Hilfe einer Zweistoffdüse besprüht. Die Zulufttemperatur betrug 60°C.

Der Luftdurchsatz wurde so gesteuert, daß sich bei einer Dosierung der Lacklösung von 50 ml/min die Produkttemperatur auf 33 bis 35°C einstellte. Die Gesamtlackmenge betrug 10 %, bezogen auf das überzogene Magnesiumsulfat. Die magensaftlöslich überzogenen sphärischen Magnesiumsulfat-Pellets waren leicht dosierbar und wiesen einen absolut neutralen Geschmack auf.

## Vergleichsversuch 3

Genauso lackierte Magnesiumsulfatpartikeln auf konventioneller Basis erwiesen sich als problematisch.

Während der Herstellung unter identischen Bedingungen mit ausgesiebtem, handelsüblichem Magnesiumsulfat mit entsprechendem Kornband traten erhebliche Agglomerationstendenzen auf. Verklebungen konnten nur durch drastische Verlangsamung des Prozeßablaufes verhindert werden. Nach Reduktion von Lacksprührate und Zulufttemperatur erhöhte sich der Zeitbedarf auf das Doppelte.

Die Lackmenge von 10 %, bezogen auf die lackierten Partikeln, reichte zudem nicht aus, um eine Geschmacksneutralität zu erzielen. Erst nach Auftragen von 15 % Lack war der bittere Magnesiumsulfat-Geschmack nicht mehr wahrzunehmen.

Die Kosten der Lackierung sind bei konventionellem Vorgehen ingesamt deutlich höher als bei sphärischem magensaftlöslich lackiertem Magnesiumsulfat gemäß vorliegender Erfindung. 50 % mehr Lackbedarf und insgesamt 3-fache Prozeßdauer lassen eine herkömmliche Herstellung vergleichsweise unrentabel erscheinen.

## Beispiel 5

In einem 6-l-Leitrohrkristallisator wie bei Beispiel 2 wurden 5 l einer auf 40°C temperierten, gesättigten Lösung von Acetylsalicylsäure in Ethanol so intensiv gerührt, daß ein stetiger vertikaler Kreislauf um das Leitrohr gewährleistet war (n = ca. 150 min⁻¹). Nach Zugabe von 5 mg Acetylsalicylsäure-Kristallkeimen mit einer Korngröße kleiner 100 μm wurde unter fortwährenden Leitrohrbedingungen mit einer Kühlrate von 4 K/h stetig auf 20°C abgekühlt.

Die entstandene Kristallsuspension wurde zur Verhinderung einer Sekundärkristallisation mit wenig Ethanol versetzt, über eine Nutsche abgesaugt und mit wenig Ethanol nachgewaschen. Die Trocknung erfolgte im Wirbelbett-Trockner bei einer Zulufttemperatur von 60°C.

Das Produkt bestand aus farblosen sphärischen Kristallen mit einem Korngrößenbereich von 0,5 bis 1,5 mm, wobei ein ausgeprägtes Maximum der Häufigkeitsverteilung bei 0,9 mm lag.

Diese sphärische Acetylsalicylsäure wurde in einem Wirbelschichtsprühgranulator kontinuierlich mit Ethylcellulose aus einer ethanolischen Lösung überzogen. Die Ethylcellulose besaß einen spezifischen Viskositätswert von 10 mPas, die Konzentration der Lösung betrug 7 % G/G. Der Polymerenlösung wurden 25 % G/G, bezogen auf das Polymerenmaterial, Dibutylphthalat als Weichmacher zugesetzt. Die Gesamtmenge des Hüllpolymeren betrug 6 % G/G, bezogen auf das überzogene Produkt.

Die Wirbelschichtlackierung wurde so gesteuert, daß die Produkttemperatur im Bereich 26 bis 28°C verweilte.

Die retardierten sphärischen Acetylsalicylsäure-Partikeln wurden mit 0,5 % hochdispersem Siliciumdioxid gemischt. Sie konnten sehr leicht und genau in üblichen Geräten in Hartgelatine-Steckkapseln abgefüllt werden.

Die Freisetzung der Acetylsalicylsäure aus den beschriebenen retardierten sphärischen Partikeln in einer Paddle-Apparatur nach USP XX (437 mg retardiertes sphärisches Produkt; Medium Wasser) betrug nach 2 Stunden 41 %.

Identisch lackierte handelsübliche Acetylsalicylsäure ergab unter gleichen Bedingungen einen Freisetzungsgrad nach 2 Stunden von 86 %.

Die sphärischen Basisteilchen ermöglichen also eine deutliche Verbesserung der Freisetzungskinetik.

**Patentansprüche** für die Vertragsstaten:
BE, CH, DE, FR, GB, IT, LI, NL, SE

1. Sphärische Einkristalle Pharmazeutischer Wirk- oder Hilfsstoffe mit Durchmessern von 0,1 bis 3 mm zum Verwendung in einem Verfahren zur therapeutischen Behandlung im menschlichen oder tierischen Körper.

2. Festes Pharmazeutisches Produkt, das mindestens einen Wirk- und einen Hilfsstoff enthält, wobei mindestens ein Wirk- oder Hilfsstoff in Form sphärischer Einkristalle vorliegt.

3. Verfahren zur Herstellung von festen pharmazeutischen Produkten durch übliches Mischen von Wirk- und Hilfsstoffen und Formen nach üblichen Methoden, dadurch gekennzeichnet, daß man dabei mindestens einen löslichen, kristallinen Wirk- oder Hilfsstoff in Form sphärischer Einkristalle mit Durchmessern von 0,1 bis 3 mm einsetzt.

**Patentansprüche** für den Vertragsstat:
AT

1. Verwendung von sphärischen Einkristallen Pharmazeutischer Wirk- oder Hilfsstoffe mit Durchmessern von 0,1 bis 3 mm zur Herstellung von festen pharmazeutischen Produkten.

2. Festes pharmazeutisches Produkt, das mindestens einen Wirk- und einen Hilfsstoff enthält, wobei mindestens ein Wirk- oder Hilfsstoff in Form sphärischer Einkristalle vorliegt.

**Claims** for the Contracting States: BE, CH, DE, FR, GB, IT, LI, NL, SE

1. Spherical single crystals of pharmaceutical active compounds or assistants, the crystals having diameters of from 0.1 to 3 mm, for use in a method for treatment in the human or animal body by therapy.

2. A solid pharmaceutical product containing one or more active compounds and one or more assistants in which one or more active compounds or assistants are present in the form of spherical single crystals.

3. A process for the preparation of a solid pharmaceutical product by conventional mixing of active compounds and assistants, and molding by conventional methods, wherein at least one soluble crystalline active compound or assistant is employed in the form of spherical single crystals having diameters of from 0.1 to 3 mm.

**Claims** for the Contracting State: AT

1. Use of spherical single crystals of pharmaceutical active compounds or assistants, the crystals having diameters of from 0.1 to 3 mm, for the preparation of solid pharmaceutical products.

2. A solid pharmaceutical product containing one or more active compounds and one or more assistants in which one or more active compounds or assistants are present in the form of spherical single crystals.

**Revendications** pour les Etats
Contractants: BE, CH, DE, FR, GB, IT, LI, NL, SE

1. Monocristaux sphériques de constituants actift ou auxiliaires pharmaceutiques de diamètres de 0,1 à 3 mm pour utilisation dans un procédé de traitement thérapeutique sur des corps humains ou animaux.

2. Produit pharmaceutique solide qui contient au moins un constituant actif et un condtituant auxiliaire, un constituant actif ou auxiliaire au moins se trouvant sous forme de monocristaux sphériques.

3. Procédé de préparation de produits pharmaceutiques solides par mélange usuel de constituants actifs et auxiliaires et mise en forme selon les méthodes uselles, caractérisé par le fait que l'on y utilise au moins constituant actif ou auxiliaire, cristallin, soluble, sous forme de monocristaux sphériques de diamètres de 0,1 à 3 mm.

**Revendications** pour l'Etat Contractant:
AT

1. Utilisation de monocristaux sphériques de constituants actifs ou auxiliares pharmaceutiques de diamètres de 0,1 à 3 mm pour la préparation de produits pharmaceutiques solides.

2. Produit pharmaceutique solide qui contient au moins un constituant actif et un constituant auxiliaire, un constituant actif ou auxiliaire au moins se trouvant sous forme de monocristaux sphériques.